# EUROPEAN PATENT SPECIFICATION

(11) **EP 2 522 348 B1**
(45) Date of publication and mention of the grant of the patent: **25.05.2016**
(21) Application number: 10839079.0
(22) Date of filing: 08.11.2010
(51) Int. Cl.: A61K 31/40, A61K 9/32, A61K 47/32, A61P 3/06

(54) **ATORVASTATIN-CONTAINING COATED PREPARATION**
ATORVASTATINHALTIGES BESCHICHTETES PRÄPARAT
PRÉPARATION ENROBÉE CONTENANT DE L'ATORVASTATINE

(30) Priority: 25.12.2009 JP 2009295316
(43) Date of publication of application: 14.11.2012
(73) Proprietor: Sawai Pharmaceutical Co., Ltd., Yodogawa-ku Osaka-shi Osaka 532-0003 (JP)
(72) Inventor: YANAGI, Toshihiro, Osaka-shi Osaka 532-0003 (JP); NOZAWA, Kenji, Osaka-shi Osaka 532-0003 (JP); MAEDA, Kazuaki, Osaka-shi Osaka 532-0003 (JP); TAKADA, Shinya, Osaka-shi Osaka 532-0003 (JP); OKAMURA, Yasufumi, Osaka-shi Osaka 532-0003 (JP)
(74) Representative: Haslam, Simon David
(86) International application number: PCT/JP2010/069815
(87) International publication number: WO 2011/077843

(56) References cited:
- EP-A1- 1 657 265
- WO-A1-2005/019286
- WO-A1-2009/028583
- WO-A2-2004/071402
- JP-A- 2007 022 941
- JP-A- 2008 506 655

## Description

### TECHNICAL FIELD

The present invention relates to an atorvastatin-containing coated preparation useful for treating hypercholesterolemia, familial hypercholesterolemia, hyperlipidemia, and like conditions.

### BACKGROUND ART

Atorvastatin has activity to selectively and competitively inhibit the HMG-CoA reductase, which is the rate-controlling enzyme of a cholesterol biosynthetic pathway, and because atorvastatin demonstrates hypocholesterolemic action, hypotriglyceridemic action, arteriosclerosis progression inhibiting action, and the like, atorvastatin is clinically used for the treatment of hypercholesterolemia, familial hypercholesterolemia, hyperlipidemia, and like conditions.

However, atorvastatin is problematic in that atorvastatin readily undergoes oxidative decomposition with oxygen present in air (see Patent Literature 1, Nonpatent Literature 1, and Nonpatent Literature 2). In the case where the active component of a pharmaceutical preparation is oxidatively decomposed, it is possible that the pharmacological effect is reduced and the safety is impaired. Accordingly, it is necessary to inhibit oxidative decomposition as much as possible.

A known method for inhibiting oxidative decomposition of atorvastatin is a method in which a pharmaceutical preparation is packaged under an atmosphere of inert gas such as nitrogen or under hypoxic conditions such as low air pressure conditions (see Patent Literature 1, Patent Literature 2, Patent Literature 3, and Patent Literature 4). However, this method requires a special instrument for filling the package with inert gas or the like when packaging. In addition, methods that inhibit oxidative decomposition by way of a packaging form allow oxidative decomposition of atorvastatin after the package is opened, and thus the stability of the pharmaceutical preparation after the package is opened is not guaranteed.

In addition, while use of an antioxidant can be envisaged as a technique for inhibiting oxidative decomposition, it has not been possible to sufficiently suppress oxidative decomposition of atorvastatin using an antioxidant.

As discussed above, regarding atorvastatin preparations, it has not been possible to obtain stable pharmaceutical preparations by conventionally known oxidative decomposition inhibitory methods.

Meanwhile, a resin composition containing a polyvinyl alcohol copolymer as a principal component useful as a coating material for medicines, veterinary medicines, agrochemicals, fertilizers, food products, and the like is known (see Patent Literature 5). Moreover, a method for stabilizing a macrolide antibiotic preparation by coating it with such a polyvinyl alcohol copolymer is known (see Patent Literature 6). However, in Patent Literature 5 and Patent Literature 6, although certain medicines are described as materials to be coated, stabilization of an atorvastatin preparation is neither described nor suggested.

### CITATION LIST

### PATENT LITERATURE

Patent Literature 1: WO2006/008091
Patent Literature 2: WO2005/011638
Patent Literature 3: WO2004/032920
Patent Literature 4: WO2007/082764
Patent Literature 5: WO2005/019286
Patent Literature 6: WO2007/126039
Patent Literature 7: WO2004/071402
Patent Literature 8: EP1657265 NONPATENTLITERATURE

Nonpatent Literature 1: Tetrahedron 49 (1993) 1979-1984
Nonpatent Literature 2: Tetrahedron 62 (2006) 7390-7395

### SUMMARY OF INVENTION

### TECHNICAL PROBLEM

An object of the present invention is to provide a stabilized atorvastatin-containing coated preparation in which oxidative decomposition of atorvastatin, a pharmacologically acceptable atorvastatin salt, or a solvate thereof is effectively inhibited.

### SOLUTION TO PROBLEM

As a result of having conducted extensive research to achieve the aforementioned object, the present inventors found that a stabilized atorvastatin-containing coated preparation can be obtained by coating a solid formulation such as an uncoated tablet or an uncoated granule containing atorvastatin, a pharmacologically acceptable atorvastatin salt, or a solvate thereof with a coating agent containing a polyvinyl alcohol copolymer. Based on this finding, the inventors conducted further research and accomplished the present invention.

The present invention provides, as described below, an
atorvastatin-containing coated preparation, a method for inhibiting generation of related substances of atorvastatin, a pharmacologically acceptable atorvastatin salt, or a solvate thereof, and a method for stabilizing atorvastatin, a pharmacologically acceptable atorvastatin salt, or a solvate thereof.

Item 1. An atorvastatin-containing coated preparation comprising a solid formulation containing atorvastatin, a pharmacologically acceptable atorvastatin salt, or a solvate thereof coated with a coating agent comprising a polyvinyl alcohol copolymer.

Item 2. The atorvastatin-containing coated preparation according to Item 1, wherein a solid formulation containing an atorvastatin calcium salt or a hydrate thereof is coated with the coating agent comprising a polyvinyl alcohol copolymer.

Item 3. The atorvastatin-containing coated preparation according to Item 1, wherein a coating amount of the coating agent comprising a polyvinyl alcohol copolymer is about 1 to about 100 parts by weight in terms of the polyvinyl alcohol copolymer relative to 100 parts by weight of the solid formulation containing atorvastatin, a pharmacologically acceptable atorvastatin salt, or a solvate thereof.

Item 4. The atorvastatin-containing coated preparation according to Item 3, wherein the coating amount of the coating agent comprising a polyvinyl alcohol copolymer is about 3 to about 50 parts by weight in terms of the polyvinyl alcohol copolymer relative to 100 parts by weight of the solid formulation containing atorvastatin, a pharmacologically acceptable atorvastatin salt, or a solvate thereof.

Item 5. The atorvastatin-containing coated preparation according to Item 4, wherein the coating amount of the coating agent comprising a polyvinyl alcohol copolymer is about 5 to about 10 parts by weight in terms of the polyvinyl alcohol copolymer relative to 100 parts by weight of the solid formulation containing atorvastatin, a pharmacologically acceptable atorvastatin salt, or a solvate thereof.

Item 6. The atorvastatin-containing coated preparation according to Item 1, wherein the solid formulation containing atorvastatin, a pharmacologically acceptable atorvastatin salt, or a solvate thereof is in an uncoated tablet or uncoated granule form.

Item 7. The atorvastatin-containing coated preparation according to Item 1, wherein the coating agent comprises a polyvinyl alcohol copolymer obtained by copolymerizing a polyvinyl alcohol having an average degree of polymerization of about 200 to about 1500 and at least one polymerizable vinyl monomer.

Item 8. The atorvastatin-containing coated preparation according to Item 7, wherein the polyvinyl alcohol is a partially saponified polyvinyl alcohol.

Item 9. The atorvastatin-containing coated preparation according to Item 7, wherein the polymerizable vinyl monomer is selected from the group consisting of unsaturated carboxylic acids, unsaturated carboxylic acid esters, unsaturated nitriles, unsaturated amides, aromatic vinyls, aliphatic vinyls, heterocycles having a unsaturated bond, and salts thereof.

Item 10. The atorvastatin-containing coated preparation according to Item 9, wherein the unsaturated carboxylic acids or salts thereof are selected from the group consisting of acrylic acid, methacrylic acid, crotonic acid, fumaric acid, maleic acid, itaconic acid, and salts thereof, and the unsaturated carboxylic acid esters are selected from the group consisting of methyl methacrylate, methyl acrylate, ethyl methacrylate, ethyl acrylate, butyl methacrylate, butyl acrylate, isobutyl methacrylate, isobutyl acrylate, cyclohexyl methacrylate, cyclohexyl acrylate, 2-ethylhexyl methacrylate, 2-ethylhexyl acrylate, hydroxyethyl methacrylate, hydroxyethyl acrylate, polyethylene glycol methacrylate, polyethylene glycol acrylate, and polypropylene glycol acrylate.

Item 11. The atorvastatin-containing coated preparation according to Item 9, wherein the unsaturated carboxylic acids, salts thereof, and unsaturated carboxylic acid esters are compounds represented by general formula (I) or salts thereof

H₂C=C(R₁)-COOR₂ (I)

wherein R₁ represents a hydrogen atom or a methyl group, and R₂ represents a hydrogen atom or a C₁-₄ alkyl group.

Item 12. The atorvastatin-containing coated preparation according to Item 10, wherein the unsaturated carboxylic acids or salts thereof are acrylic acid or salts thereof, and the unsaturated carboxylic acid esters are methyl methacrylate.

Item 13. The atorvastatin-containing coated preparation according to Item 7, wherein the polyvinyl alcohol copolymer is obtained by copolymerizing a partially saponified polyvinyl alcohol having an average degree of polymerization of about 300 to about 500 and a polymerizable vinyl monomer in a weight ratio of about 6 : 4 to about 9 : 1, the polymerizable vinyl monomer refers to acrylic acid and methyl methacrylate, and the polymerizable vinyl monomer has a weight ratio of about 3 : 7 to about 0.5 : 9.5 when being subjected to copolymerization.

Item 14. A method for inhibiting generation of related substances of atorvastatin, a pharmacologically acceptable atorvastatin salt, or a solvate thereof, the method comprising coating a solid formulation containing atorvastatin, a pharmacologically acceptable atorvastatin salt, or a solvate thereof with a coating agent comprising a polyvinyl alcohol copolymer.

Item 15. A method for stabilizing atorvastatin, a pharmacologically acceptable atorvastatin salt, or a solvate thereof, the method comprising coating a solid formulation containing atorvastatin, a pharmacologically acceptable atorvastatin salt, or a solvate thereof with a coating agent comprising a polyvinyl alcohol copolymer.

### ADVANTAGEOUS EFFECTS OF INVENTION

According to the present invention, the following remarkable effects can be obtained with a solid formulation containing atorvastatin, a pharmacologically acceptable atorvastatin salt, or a solvate thereof coated with a coating agent containing a polyvinyl alcohol copolymer.
(1) Oxidative decomposition of atorvastatin, a pharmacologically acceptable atorvastatin salt, or a solvate thereof in an atorvastatin preparation is effectively inhibited over a long period of time, thus allowing a stabilized atorvastatin-containing coated preparation to be obtained. Moreover, a method for inhibiting generation of related substances of atorvastatin, a pharmacologically acceptable atorvastatin salt, or a solvate thereof, and a method for stabilizing atorvastatin, a pharmacologically acceptable atorvastatin salt, or a solvate thereof are provided.
(2) Oxidative decomposition of the resulting atorvastatin-containing coated preparation is inhibited and, therefore, impurities such as related substances or the like of atorvastatin, a pharmacologically acceptable atorvastatin salt, or a solvate thereof are barely present, and deterioration of pharmaceutical effects and impairment of safety can be prevented over a long period of time.
(3) A stabilized atorvastatin-containing coated preparation can be readily prepared by a simple method in which a solid formulation containing atorvastatin, a pharmacologically acceptable atorvastatin salt, or a solvate thereof is coated with a coating agent containing a polyvinyl alcohol copolymer.
(4) Even when the solid formulation containing atorvastatin, a pharmacologically acceptable atorvastatin salt, or a solvate thereof is coated with the coating agent containing a polyvinyl alcohol copolymer, the solubility of the main ingredient, i.e., atorvastatin, a pharmacologically acceptable atorvastatin salt, or a solvate thereof, is not inhibited.

### BRIEF DESCRIPTION OF DRAWINGS

[Fig. 1] Fig. 1 is a graph showing results of a dissolution test of the atorvastatin-containing coated tablets obtained in Example 2, Example 3, and Comparative Example 1.
[Fig. 2] Fig. 2 is a bar graph showing results of a stability test carried out on the atorvastatin-containing coated tablets obtained in Example 4, Example 5, Example 6, and Comparative Example 2 under the high temperatures.
[Fig. 3] Fig. 3 is a bar graph showing results of a stability test carried out on of the atorvastatin-containing coated tablets obtained in Example 4, Example 5, Example 6, and Comparative Example 2 under the high temperatures and high humidities.

### DESCRIPTION OF EMBODIMENTS

### Atorvastatin-containing coated preparation

In the atorvastatin-containing coated preparation of the present invention, a solid formulation containing atorvastatin, a pharmacologically acceptable atorvastatin salt, or a solvate thereof is coated with a coating agent containing a polyvinyl alcohol copolymer.

### Solid formulation containing atorvastatin, pharmacologically acceptable atorvastatin salt, or solvate thereof

Uncoated tablets, uncoated granules, and the like that are composed of atorvastatin, a pharmacologically acceptable atorvastatin salt, or a solvate thereof, and at least one excipient selected from the group consisting of diluents, disintegrators, and binders are preferably encompassed within the aforementioned solid formulation.

### Atorvastatin

Atorvastatin is an active pharmaceutical ingredient of the coated preparation of the present invention, and the chemical name thereof is [R-(R*,R*)]-2-(4-fluorophenyl)-β,δ-dihydroxy-5-(1-methylethyl)-3-phenyl-4-[(phenylami no)carbonyl]-1H-pyrrole-1-heptanoic acid. As stated above, atorvastatin has activity of inhibiting the 3-hydroxy-3-methylglutaryl coenzyme A (HMG-CoA).

As pharmacologically acceptable atorvastatin salts, a calcium salt, a magnesium salt, a sodium salt, a potassium salt, an aluminium salt, and the like are usable. As solvates thereof, a hydrate, an acetonic solvate, a methanolic solvate, an ethanolic solvate, and the like are usable. These salts or solvates thereof may be amorphous or crystalline. It is particularly preferable to use atorvastatin calcium trihydrate. The atorvastatin calcium trihydrate has a chemical name, (-)-monocalcium
bis{(3R,5R)-7-[2-(4-fluorophenyl)-5-isopropyl-3-phenyl-4-phenylcarbamoyl-1H-pyrrol-1-yl]-3,5-dihydroxyheptanoate} trihydrate.

The atorvastatin-containing coated preparation of the present invention may contain other active pharmaceutical agents as necessary. Usable other active pharmaceutical agents include captopril, enalapril, lisinopril, benazepril, irbesartan, losartan, valsartan, candesartan, telmisartan, olmesartan, amlodipine besylate, nifedipine, atenolol, carvedilol, betaxolol, pharmacologically acceptable salts thereof, and solvates thereof.

### Excipients

Atorvastatin, a pharmacologically acceptable atorvastatin salt, or a solvate thereof is usually used in combination with pharmacologically acceptable excipients such as a diluent, a disintegrator, a binder, and the like for enhancing fluidity and adhesion.

Examples of diluents include crystalline cellulose, starch such as corn starch; lactose, powdered sugar, granulated sugar, glucose, mannitol, light anhydrous silicic acid, talc, magnesium oxide, magnesium carbonate, calcium carbonate, and the like. These diluents can be used singly or as a combination of two or more.

Examples of disintegrators include carmellose calcium (for example, ECG505, manufactured by Nichirin Chemical Co., Ltd.), carmellose sodium, croscarmellose sodium (for example, Ac-Di-Sol, manufactured by FMC), crosslinked polyvinyl pyrrolidone (for example, Kollidon CL, manufactured by BASF), low-substituted hydroxypropylcellulose (for example, L-HPC, manufactured by Shin-Etsu Chemical Co., Ltd.), starches, and the like. These disintegrators can be used singly or as a mixture of two or more.

Examples of binders include conventional binders such as sucrose, gelatin, powdered gum arabic, methylcellulose, hydroxypropylmethylcellulose, hydroxypropylcellulose (for example, HPC-L, manufactured by Nippon Soda Co., Ltd.), carboxymethylcellulose (carmellose), crystalline cellulose-sodium carboxymethylcellulose (for example, Avicel RC, manufactured by Asahi Kasei Corporation), polyvinyl pyrrolidone, pullulan, dextrin, tragacanth, sodium alginate, pregelatinized starch, and the like. These binders can be used singly or as a mixture of two or more.

The proportion of these excipients can be selected from the range of about 200 to about 3000 parts by weight relative to 100 parts by weight of atorvastatin, a pharmacologically acceptable atorvastatin salt, or a solvate thereof, and usually it is preferably about 300 to about 2500 parts by weight, and more preferably about 500 to about 2000 parts by weight.

In addition to pharmacologically acceptable excipients such as diluents, disintegrators, and binders, other pharmacologically acceptable commonly used excipients such as lubricants, fluidizers, antistatic agents, surfactants, colorants, corrigents, wetting agents, fillers, bulking agents, adsorbents, preservatives (for example, antiseptics), buffers, and disintegration extending agents may be added to atorvastatin, a pharmacologically acceptable atorvastatin salt, or a solvate thereof.

Examples of the aforementioned lubricants include magnesium stearate, talc, stearic acid, calcium stearate, and the like. Examples of the antistatic agents include light anhydrous silicic acid and the like. Examples of the surfactants include anionic surfactants such as sodium alkylsulfate; nonionic surfactants such as polyoxyethylene sorbitan fatty acid esters, polyoxyethylene fatty acid esters, and polyoxyethylene castor oil derivatives; and the like. Examples of the colorants include tar dye, caramel color, red iron oxide, titanium oxide, and the like. Examples of the corrigents include sweeteners such as sucrose, lactose, mannitol, xylitol, saccharin, saccharin sodium, aspartame, and stevioside; flavoring agents; and the like. Examples of the wetting agents include polyethylene glycol (macrogol), glycerol, propylene glycol, and the like.

These other commonly used excipients can be used singly or as a mixture of two or more. The amounts of these ingredients contained in the final pharmaceutical preparation are not particularly limited.

In the present invention, a solid formulation such as an uncoated tablet or an uncoated granule containing atorvastatin, a pharmacologically acceptable atorvastatin salt, or a solvate thereof can be produced according to a method known in the pharmaceutical field. For example, an uncoated tablet can be produced by performing mixing, granulation, drying, particle size regulation, tableting, and like operations on atorvastatin, a pharmacologically acceptable atorvastatin salt, or a solvate thereof, and excipients such as a diluent, a disintegrator, and a binder, using a usual solvent according to a method well-known in the art. A disintegrator, a lubricant, and the like may be added after particle size regulation and before tableting. Regarding these operations, uncoated granules can be produced by performing the operations as far as the particle size regulation operation. Among these operations, the granulation operation may be performed using a device such as an agitation granulator, a fluidized-bed granulator, a Brabender, or a Twin dome granulator, but it is preferable to use an agitation granulator. Tableting can be performed using a commercially available tableting machine.

### Coating agent containing polyvinyl alcohol copolymer

As the coating agent containing a polyvinyl alcohol copolymer for use in the present invention, it is preferable to use a coating agent composed of the polyvinyl alcohol copolymer described in WO2005/019286 blended with, if necessary, a lubricant, a colorant, a plasticizer, or the like.

The polyvinyl alcohol copolymer used as the main ingredient in the coating composition for use in the present invention can be produced by copolymerizing a polyvinyl alcohol or a derivative (for example, an ester) or salt thereof with at least one polymerizable vinyl monomer by a method known per se.

Examples of methods for producing such a polyvinyl alcohol copolymer include radical polymerization, for example, solution polymerization, suspension polymerization, emulsion polymerization, bulk polymerization, and like methods that are known per se, and these methods can be performed under usual polymerization conditions. The polymerization reaction is usually performed in water, an organic solvent, or a mixture thereof in the presence of a polymerization initiator and, if necessary, in the presence of a reducing agent, a chain transfer agent, a dispersant, or the like. Removal of an unreacted monomer, drying, pulverization, and the like may be performed according to known methods, and are not particularly limited.

Examples of the reducing agent include sodium erythorbate, sodium metabisulfite, ascorbic acid, and the like. Examples of the chain transfer agent include 2-mercaptoethanol, α-methylstyrene dimer, 2-ethylhexyl thioglycolate, lauryl mercaptan, and the like. Examples of the dispersant include surfactants such as sorbitan ester and lauryl alcohol, and the like. Examples of the organic solvent include methanol, ethanol, cellosolve, carbitol, and the like.

It is usually preferable that the polyvinyl alcohol that serves an ingredient of the polyvinyl alcohol copolymer of the present invention has an average degree of polymerization of about 200 to about 1500, more preferably about 200 to about 1300, even more preferably about 200 to about 900, particularly more preferably about 200 to about 600, and most preferably about 300 to about 500. As the polyvinyl alcohol, a partially saponified polyvinyl alcohol is preferable. The degree of saponification of the polyvinyl alcohol is preferably about 60 to about 100 mol%, and more preferably about 78 to about 96 mol%. The saponified polyvinyl alcohol can be produced by subjecting vinyl acetate to radical polymerization and suitably saponifying the resulting vinyl acetate resin. Production of the desired polyvinyl alcohol can be achieved by suitably controlling the degree of polymerization and the degree of saponification according to methods known per se.

A commercially available product can be used as the partially saponified polyvinyl alcohol. Preferable examples of commercially available polyvinyl alcohols include Gohsenol EG05 (manufactured by Nippon Synthetic Chemical Industry Co., Ltd.), Gohsenol EG25 (manufactured by Nippon Synthetic Chemical Industry Co., Ltd.), PVA203 (manufactured by Kuraray Co., Ltd.), PVA204 (manufactured by Kuraray Co., Ltd.), PVA205 (manufactured by Kuraray Co., Ltd.), JP-04 (manufactured by Japan Vam & Poval Co., Ltd.), JP-05 (manufactured by Japan Vam & Poval Co., Ltd.), and the like. In the production of the polyvinyl alcohol copolymer that serve as the principal component of the coating agent for use in the present invention, not only a single polyvinyl alcohol is used as an ingredient, but also two or more polyvinyl alcohols having different degrees of polymerization or degrees of saponification can be suitably used in combination depending on the purpose. For example, a polyvinyl alcohol having an average degree of polymerization of 300 and a polyvinyl alcohol having an average degree of polymerization of 1500 can be used as a mixture.

As the polyvinyl alcohol for use as an ingredient, a variety of modified polyvinyl alcohols can be used therefor. Examples include amine-modified polyvinyl alcohols, ethylene-modified polyvinyl alcohols, carboxylic acid-modified polyvinyl alcohols, diacetone-modified polyvinyl alcohols, thiol-modified polyvinyl alcohols, and the like. For these modified polyvinyl alcohols, commercially-available products or those produced according to methods known in the art can be used.

Examples of the polymerizable vinyl monomer to be polymerized with the ingredient polyvinyl alcohol include unsaturated carboxylic acids, unsaturated carboxylic acid esters, unsaturated nitriles, unsaturated amides, aromatic vinyls, aliphatic vinyls, unsaturated bond-containing heterocycles, salts thereof, and the like. Examples of unsaturated carboxylic acids and salts thereof include acrylic acid, methacrylic acid, crotonic acid, fumaric acid, maleic acid, itaconic acid, and like unsaturated carboxylic acids or salts thereof (such as alkali metal salts, ammonium salts, and alkylamine salts). Examples of unsaturated carboxylic acid esters include substituted or unsubstituted alkyl esters, cyclic alkyl esters, polyalkylene glycol esters, and the like.

Specifically, (1) examples of acrylic acid esters include methyl acrylate, ethyl acrylate, butyl acrylate, isobutyl acrylate, cyclohexyl acrylate, 2-ethylhexyl acrylate, hydroxyethyl acrylate, polyethylene glycol acrylate, polypropylene glycol acrylate, and the like, (2) examples of methacrylic acid esters include methyl methacrylate, ethyl methacrylate, butyl methacrylate, isobutyl methacrylate, cyclohexyl methacrylate, 2-ethylhexyl methacrylate, hydroxyethyl methacrylate, polyethylene glycol methacrylate, and the like, (3) examples of unsaturated nitriles include acrylonitrile, methacrylonitrile, and the like, (4) examples of unsaturated amides include acrylamide, dimethylacrylamide, methacrylamide, and the like, (5) examples of aromatic vinyls include styrene, α-methyl styrene, and the like, (6) examples of aliphatic vinyls include vinyl acetate and the like, and (7) examples of unsaturated bond-containing heterocycles include N-vinyl pyrrolidone, acryloyl morpholine, and the like.

Unsaturated carboxylic acids, salts thereof, and unsaturated carboxylic acid esters, each serving as the polymerizable vinyl monomer when producing the polyvinyl alcohol copolymer, are represented by the following formula (I):

H₂C=C(R₁)-COOR₂ (I)

(wherein R₁ represents a hydrogen atom or a methyl group, and R₂ represents a hydrogen atom or a C₁-₄ alkyl group) or salts thereof are preferably used.

These polymerizable vinyl monomers can be used singly or two or more can be used as a combination for copolymerization with the polyvinyl alcohol. It is preferable that a mixture of acrylic acid and a methacrylic acid ester (such as methyl methacrylate) is copolymerized with the polyvinyl alcohol. The weight ratio of the polyvinyl alcohol to the polymerizable vinyl monomer herein is about 6 : 4 to about 9 : 1, and preferably about 8 : 2. In the case where acrylic acid and methyl methacrylate are used as polymerizable vinyl monomers, the weight ratio thereof is preferably about 3 : 7 to about 0.5 : 9.5, and particularly preferably about 1.25 : 8.75. A preferable polyvinyl alcohol copolymer for use as the principal component of the coating agent is a copolymer composed of a polyvinyl alcohol (having an average degree of polymerization of about 200 to about 1300), methyl methacrylate, and acrylic acid, with the weight ratio thereof being about 60 to 90 : 7 to 38 : 0.5 to 12, and a particularly preferable copolymer has a weight ratio of about 80 : 17.5 : 2.5.

Known polymerization initiators can be used. Specific examples include inorganic peroxides such as potassium persulfate, ammonium persulfate, and hydrogen peroxide; organic peroxides such as peracetic acid, tert-butyl hydroperoxide, and di-n-propyl peroxydicarbonate; azo compounds such as
2,2'-azobis(2-amidinopropane)hydrochloride and 2,2'-azobis(2,4-dimethylvaleronitrile); and the like.

The coating agent used in the present invention can take various forms. Generally, the coating agent may be used in the form of an aqueous solution, an aqueous dispersion, an organic solvent solution, or an organic solvent dispersion of a polyvinyl alcohol copolymer blended with, if necessary, a lubricant, a colorant, a plasticizer, or the like. Here, it is usually preferable that the polyvinyl alcohol copolymer is contained in the coating agent in an amount of about 10 to about 100 wt%, and more preferably about 30 to about 70 wt%. As lubricants, colorants, plasticizers, and the like that are blended if necessary, those identical to the components exemplified above for the aforementioned uncoated tablet can be used.

While the principal component of the coating agent is a polyvinyl alcohol copolymer, the coating agent may contain other coating components. As other coating components, hydroxypropylmethylcellulose, hydroxypropylcellulose, methylcellulose, polyvinyl alcohol, polyvinyl pyrrolidone, polyethylene glycol, sucrose, and like coating materials well known in the pharmaceutical field are usable.

In the case where the coating agent is subjected to coating an uncoated tablet, an uncoated granule, and the like, it is preferable to perform coating using a film coating machine, a fluidized-bed granulator, and like means.

The amount of the coating agent for coating is preferably within the range of about 1 to about 100 parts by weight, more preferably about 3 to about 50 parts by weight, and even more preferably about 5 to about 10 parts by weight in terms of polyvinyl alcohol copolymer relative to 100 parts by weight of a solid formulation such as an uncoated tablet or an uncoated granule containing atorvastatin, a pharmacologically acceptable atorvastatin salt, or a solvate thereof.

When the coating agent is subjected to coating an uncoated tablet, an uncoated granule, or the like, such an uncoated tablet, an uncoated granule, or the like may be pre-coated in advance, or a pharmaceutical preparation that has been coated with the coating agent may be over-coated. For pre-coating and over-coating, for example, hydroxypropylmethylcellulose, hydroxypropylcellulose, methylcellulose, polyvinyl alcohol, polyvinyl pyrrolidone, polyethylene glycol, sucrose, and like coating materials well known in this field can be used.

The atorvastatin-containing coated pharmaceutical preparation of the present invention can be used in various forms (for example, tablets, granules, pills, capsules, dry syrups, and the like), and is preferably used in a tablet form.

When using the atorvastatin-containing coated pharmaceutical preparation of the present invention, it is sufficient that the pharmaceutical preparation is administered to a human in an amount effective to treat a disease such hypercholesterolemia, familial hypercholesterolemia, or hyperlipidemia. The dosage may be different depending on the age, body weight, symptom, sex, or a like feature of the patient, but usually the pharmaceutical preparation can be orally administered once or several times in an amount of, for example, about 5 to about 40 mg in terms of atorvastatin per day.

Regarding the coated pharmaceutical preparation of the present invention after storage, for example, at 60°C at 60% RH for 2 weeks, the total amount of related substance 2.5 % or less and more preferably 2% or less. Here, examples of related substances include defluorinated compounds, difluoro compounds, diastereomers, lactonic compounds, oxidatively decomposed products, and the like.

Preferably, the coated pharmaceutical preparation of the present invention may be press-through packaged (PTP) or bottled (for example, plastic-bottled, glass-bottled, aluminium-canned) to further enhance stability A deodorizer, a desiccant, a deoxidizer, and the like may be included in the package.

The present invention provides a method for inhibiting generation of related substances of atorvastatin, a pharmacologically acceptable atorvastatin salt, or a solvate thereof, which is characterized by coating a solid formulation containing atorvastatin, a pharmacologically acceptable atorvastatin salt, or a solvate thereof with a coating agent containing a polyvinyl alcohol copolymer. Atorvastatin, a pharmacologically acceptable atorvastatin salt, or a solvate thereof as well as a coating agent containing a polyvinyl alcohol copolymer, a coating method, and the like are as described above.

Also, the present invention provides a method for stabilizing atorvastatin, a pharmacologically acceptable atorvastatin salt, or a solvate thereof, which is characterized by coating a solid formulation comprising atorvastatin, a pharmacologically acceptable atorvastatin salt, or a solvate thereof with a coating agent containing a polyvinyl alcohol copolymer. Atorvastatin, a pharmacologically acceptable atorvastatin salt, or a solvate thereof as well as a coating agent containing a polyvinyl alcohol copolymer, a coating method, and the like are as described above.

### EXAMPLES

The present invention shall be described in more detail by way of examples and comparative examples, but the present invention is not limited to the examples in any way.

### Examples 1 to 3 and Comparative Example 1

### (1) Preparation of uncoated tablets

933 g of microcrystalline cellulose, 750 g of carmellose calcium, and 1500 g of magnesium oxide were charged into a high-speed mixer granulator (trade name "High-Speed Mixer FS-GS-10" manufactured by Fukae Powtec), and mixed for 1 minute. Then, a solution in which 542 g of atorvastatin calcium trihydrate had been dissolved in 1007 g of an 9/1 (weight ratio) mixture of dichloromethane/ethanol was introduced, and granulation was carried out for 4 minutes. The resulting granules were introduced into a fluidized-bed dryer (trade name "FLO-5" manufactured by Freund Corporation), and dried until a discharge air temperature of 45°C was attained with an intake air temperature of 60°C. The resulting dried product was passed through a 1.0 mm screen in a particle size regulator (trade name "Power Mill P-04S" manufactured by Dalton Co., Ltd.) to give a particle size-regulated product. To the resulting product were added 250 g of carmellose calcium and 25 g of magnesium stearate, and mixed for 3 minutes with a mixer (trade name "V Blender VM-30" manufactured by Fuji Paudal Co., Ltd.). The resulting mixture was tableted with a rotary tableting machine (trade name "VIRGO-512" manufactured by Kikusui Seisakusho Ltd.) so as to attain a weight of 80 mg, a diameter of 6.0 mm, and a thickness of 2.50 mm per tablet.

The formulation per uncoated tablet was as follows:

| | |
|---|---|
| Atorvastatin calcium trihydrate | 10.84 mg |
| Microcrystalline cellulose | 18.66 mg |
| Carmellose calcium | 15 mg |
| Magnesium oxide | 30 mg |
| Carmellose calcium | 5 mg |
| Magnesium stearate | 0.5 mg |

### (2) Coating for uncoated tablet

45 parts of a polyvinyl alcohol copolymer (trade name "Povacoat Type F" (registered trademark) partially saponified polyvinyl alcohol-acrylic-methyl methacrylate copolymer, manufactured by Nisshin Kasei Co., Ltd.), 45 parts of talc, and 10 parts of titanium oxide were dissolved or dispersed in 500 parts of a 1/9 (weight ratio) mixture of ethanol/purified water to give a coating fluid. Uncoated tablets obtained in (1) above were coated with this coating fluid in a film coating machine (trade name "Dria Coater DRC500/650" manufactured by Powrex Corporation) so as to attain a weight of 88 mg, 90 mg, and 92 mg per tablet to give atorvastatin-containing coated tablets of Examples 1, 2, and 3, respectively.

In contrast, 22 parts of hydroxypropylmethylcellulose (hereinafter referred to as "HPMC") (trade name "TC-5" manufactured by Shin-Etsu Chemical Co., Ltd.), 2 parts of Polyethylene Glycol 6000 (hereinafter "PEG6000"), 1 part of talc, and 2 parts of titanium oxide were dissolved or dispersed in 200 parts of purified water to give a coating fluid. Uncoated tablets obtained in (1) above were coated with this coating fluid in a film coating machine (trade name "Hi-Coater HC-LABO" manufactured by Freund Corporation) so as to attain a weight of 90 mg per tablet to give atorvastatin-containing coated tablets of Comparative Example 1.

The formulation of the coated portion is shown in Table 1.

**Table 1**

| | Ex. 1 | Ex. 2 | Ex. 3 | Comp. Ex. 1 |
|---|---|---|---|---|
| Povacoat Type F | 3.6 | 4.5 | 5.4 | |
| Talc | 3.6 | 4.5 | 5.4 | 0.37 |
| Titanium oxide | 0.8 | 1.0 | 1.2 | 0.74 |
| PEG6000 | | | | 0.74 |
| HPMC | | | | 8.15 |
| Coating amount (mg) | 8 | 10 | 12 | 10 |
| Amount of coated tablet (mg) | 88 | 90 | 92 | 90 |

Next, the atorvastatin-containing coated tablets obtained in Example 2, Example 3, and Comparative Example 1 were subjected to a dissolution test. The methods of the dissolution test and HPLC measurement are as follows.

### Dissolution test

A test was carried out according to the Japanese Pharmacopoeia, 14th Edition, Dissolution Test, 2nd Method (Paddle Method), and the rate of dissolution after 5, 10, and 15 minutes was measured using HPLC.
Amount of each sample: 1 tablet
Test fluid : purified water
Paddle speed: 50 rpm

### HPLC measurement

Detector: ultraviolet absorptiometer (measurement wavelength: 242 nm)
Column: "Inertsil ODS-3" (particle size of 5 µm, column size of 3.0×150 mm, manufactured by GL Sciences Inc.)
Column temperature: constant temperature near 40°C
Mobile phase: 0.025 mol/L of phosphate buffer (pH 4.5) / methanol mixture (3 : 7 (volume ratio))
Flow rate: adjusted so as to attain an atorvastatin retention time of about 3 minutes.

Results of the dissolution test are shown in Table 2.

**Table 2**

| | | Rate of dissolution (%) | | |
|---|---|---|---|---|
| Time (min) | 0 | 5 | 10 | 15 |
| Ex. 2 | 0 | 62.8 | 89.7 | 98.1 |
| Ex. 3 | 0 | 52.1 | 88.2 | 100.3 |
| Comp. Ex. 1 | 0 | 51.1 | 88.8 | 99.9 |

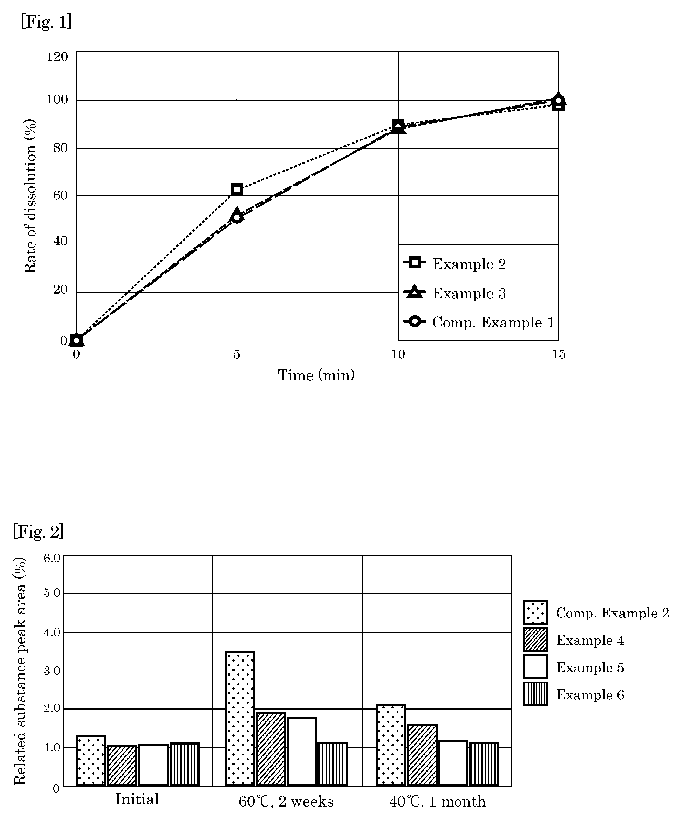

Fig. 1 is a graph showing the results of a dissolution test of the atorvastatin-containing coated tablets obtained in Example 2, Example 3, and Comparative Example 1.

It is clear from Table 2 and Fig. 1 that even when the atorvastatin calcium-containing uncoated tablets are coated with a coating agent containing a polyvinyl alcohol copolymer, the solubility of the main ingredient, i.e., atorvastatin, is not inhibited.

The atorvastatin-containing coated tablets obtained in Example 2, Example 3, and Comparative Example 1 were subjected to a stability test. The stability test was carried out under storage conditions of (1) 60°C and (2) 60°C and 60% RH.

Of the coated tablets at the beginning and at the end of each stability test, the atorvastatin peak area (%) was measured using HPLC. The measuring method is as follows. Below, measurement of a peak area in the stability tests was performed in the identical manner.

### HPLC measurement

Detector: ultraviolet absorptiometer (measurement wavelength: 254 nm)
Column: "Inertsil ODS-3" (particle size of 5 µm, column size of 4.6×250 mm, manufactured by GL Sciences Inc.)
Column temperature: constant temperature near 35°C
Mobile phase: 0.025 mol/L of phosphate buffer (pH 4.5) / acetonitrile / tetrahydrofuran mixture (10 : 12 : 3 (volume ratio))
Flow rate: adjusted so as to attain an atorvastatin retention time of about 9 minutes.
Range of area measured: range 4 times as large as the range indicating the atorvastatin retention time.

Table 3 shows atorvastatin peak areas (%).

**Table 3**

| | | Atorvastatin peak area (%) | | | |
|---|---|---|---|---|---|
| | Initial | 60°C | | 60°C, 60% RH | |
| | | For 8 days | For 2 weeks | For 8 days | For 2 weeks |
| Ex. 1 | 99.56 | 98.66 | 97.67 | 98.72 | 97.84 |
| Ex. 2 | 99.56 | 99.47 | 98.75 | 99.22 | 99.06 |
| Ex. 3 | 99.56 | 99.25 | 99.27 | 99.48 | 99.01 |
| Comp. Ex. 1 | 99.41 | 98.07 | 97.44 | 98.39 | 97.28 |

Table 4 shows the total related substance peak areas (%) calculated by subtracting an atorvastatin peak area (%) from 100 ("100 - atorvastatin peak area (%)").

**Table 4**

| | | Total related substance peak area (%) | | | |
|---|---|---|---|---|---|
| | Initial | 60°C | | 60°C, 60% RH | |
| | | For 8 days | For 2 weeks | For 8 days | For 2 weeks |
| Ex. 1 | 0.44 | 1.34 | 2.33 | 1.28 | 2.16 |
| Ex. 2 | 0.44 | 0.53 | 1.25 | 0.78 | 0.94 |
| Ex. 3 | 0.44 | 0.75 | 0.73 | 0.52 | 0.99 |
| Comp. Ex. 1 | 0.59 | 1.93 | 2.56 | 1.61 | 2.72 |

It is clear from Table 3 and Table 4 that generation of related substances is inhibited by coating atorvastatin calcium-containing uncoated tablets with a coating agent containing a polyvinyl alcohol copolymer, and stability is significantly enhanced.

### Examples 4 to 6 and Comparative Example 2

### (1) Preparation of uncoated tablets

111.44 g of microcrystalline cellulose, 20 g of croscarmellose sodium, and 120 g of magnesium oxide were charged into a high-speed mixer granulator (trade name "High-Speed Mixer LFS-GS-2J" manufactured by Fukae Powtec), and mixed for 1 minute. Then, a solution in which 43.36 g of atorvastatin calcium trihydrate and 3.20 g of polyoxyethylene sorbitan monooleate (trade name "Polysorbate 80" manufactured by Nihon Surfactant Kogyo K.K., Inc.) had been dissolved in 97.97 g of an 9/1 (weight ratio) mixture of dichloromethane/ethanol was introduced, and granulation was carried out for 4 minutes. The resulting granules were dried at 50°C for 3 hours by a tray dryer "Mini Jet Oven MO-921" manufactured by Toyama Sangyo Co., Ltd.). The resulting dried product was subjected to particle size regulation using a No. 22 sieve to give a particle size-regulated product. To the resulting product were added 40 g of croscarmellose sodium and 2 g of magnesium stearate, and mixed for 3 minutes with a mixer (trade name "V Blender VM-5" manufactured by Fuji Paudal Co., Ltd.). The resulting mixture was tableted with a single-punch tableting machine (No. 2B manufactured by Kikusui Seisakusho Ltd.) so as to attain a weight of 85 mg, a diameter of 6.0 mm, and a thickness of 2.55 mm per tablet.

The formulation per uncoated tablet was as follows:

| | |
|---|---|
| Atorvastatin calcium trihydrate | 10.84 mg |
| Polyoxyethylene sorbitan monooleate | 0.8 mg |
| Microcrystalline cellulose | 27.86 mg |
| Croscarmellose calcium | 5 mg |
| Magnesium oxide | 30 mg |
| Croscarmellose calcium | 10 mg |
| Magnesium stearate | 0.5 mg |

### (2) Coating for uncoated tablet

20 parts of a polyvinyl alcohol copolymer (trade name "Povacoat Type F" (registered trademark) manufactured by Nisshin Kasei Co., Ltd.), 20 parts of talc, and 5 parts of titanium oxide were dissolved or dispersed in 250 parts of a 1/9 (weight ratio) mixture of ethanol/purified water to give a coating fluid. Uncoated tablets obtained in (1) above were coated with this coating fluid in a film coating machine (trade name "Hi-Coater HC-LABO" manufactured by Freund Corporation) so as to attain a weight of 90 mg, 95 mg, and 100 mg per tablet to give atorvastatin-containing coated tablets of Examples 4, 5, and 6, respectively.

In contrast, 22 parts of HPMC (trade name "TC-5" manufactured by Shin-Etsu Chemical Co., Ltd.), 2 parts of PEG6000, 1 part of talc, and 2 parts of titanium oxide were dissolved or dispersed in 200 parts of purified water to give a coating fluid. Uncoated tablets obtained in (1) above were coated with this coating fluid in a film coating machine (trade name "Hi-Coater HC-LABO" manufactured by Freund Corporation) so as to attain a weight of 88 mg per tablet to give atorvastatin-containing coated tablets of Comparative Example 2.

The formulation of the coated portion is shown in Table 5.

**Table 5**

| | Ex. 4 | Ex. 5 | Ex. 6 | Comp. Ex. 2 |
|---|---|---|---|---|
| Povacoat Type F | 2.22 | 4.44 | 6.66 | |
| Talc | 2.22 | 4.44 | 6.66 | 0.11 |
| Titanium oxide | 0.56 | 1.12 | 1.68 | 0.22 |
| PEG6000 | | | | 0.22 |
| HPMC | | | | 2.45 |
| Coating amount (mg) | 5 | 10 | 15 | 3 |
| Amount of coated tablet (mg) | 90 | 95 | 100 | 88 |

The atorvastatin-containing coated tablets obtained in Example 4, Example 5, Example 6, and Comparative Example 2 were subjected to a stability test. The stability test was carried out under storage conditions of (1) 60°C, (2) 40°C, (3) 60°C and 60% RH, and (4) 40°C and 75% RH.

Of the coated tablets at the initial and at the end of each stability test, the total related substances peak area (%) was measured using HPLC. Table 6 shows the results.

**Table 6**

| | | Total related substance peak area (%) | | | |
|---|---|---|---|---|---|
| | Initial | 60°C, for 2 weeks | 40°C, for 1 month | 60°C, 60% RH, for 2 weeks | 40°C, 75% RH, for 1 month |
| Ex. 4 | 1.041 | 1.898 | 1.576 | 2.338 | 2.164 |
| Ex. 5 | 1.059 | 1.769 | 1.172 | 1.714 | 1.132 |
| Ex. 6 | 1.109 | 1.122 | 1.129 | 1.187 | 1.278 |
| Comp. Ex. 2 | 1.305 | 3.476 | 2.108 | 3.140 | 2.411 |

Also, Fig. 2 is a bar graph showing the results of measuring the total related substance peak areas (%) at the beginning of storage and after storage under conditions of (1) 60°C for 2 weeks and (2) 40°C for 1 month.

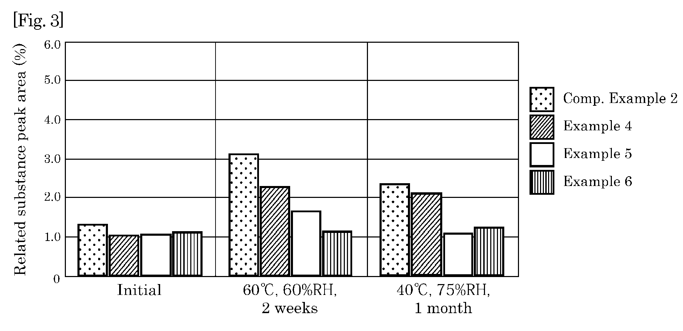

Fig. 3 is a bar graph showing the results of measuring the total related substance peak areas (%) at the beginning of storage and after storage under conditions of (3) 60°C and 60% RH for 2 weeks and (4) 40°C and 75% RH for 1 month.

It is clear from Table 6, Figure 2, and Figure 3 that generation of related substances is inhibited by coating atorvastatin calcium-containing uncoated tablets with a coating agent containing a polyvinyl alcohol copolymer, and stability is significantly enhanced.

### INDUSTRIAL APPLICABILITY

The atorvastatin-containing coated preparation of the present invention is usable as a medicine useful for treatment of patients of hypercholesterolemia, familial hypercholesterolemia, hyperlipidemia, and like diseases. Oxidative decomposition of atorvastatin, a pharmacologically acceptable atorvastatin salt, or a solvate thereof present in the atorvastatin-containing coated preparation is effectively inhibited, and thus the preparation is useful as a stabilized atorvastatin-containing coated preparation. Accordingly, the present invention is effectively used in the pharmaceutical field.

## Claims

1. An atorvastatin-containing coated preparation comprising a solid formulation containing atorvastatin, a pharmacologically acceptable atorvastatin salt, or a solvate thereof, coated with a coating agent comprising a polyvinyl alcohol copolymer.

2. The atorvastatin-containing coated preparation according to claim 1, wherein a solid formulation containing atorvastatin calcium or a hydrate thereof is coated with the coating agent comprising a polyvinyl alcohol copolymer.

3. The atorvastatin-containing coated preparation according to claim 1, wherein a coating amount of the coating agent comprising a polyvinyl alcohol copolymer is 1 to 100 parts by weight in terms of the polyvinyl alcohol copolymer relative to 100 parts by weight of the solid formulation containing atorvastatin, a pharmacologically acceptable atorvastatin salt, or a solvate thereof.

4. The atorvastatin-containing coated preparation according to claim 3, wherein the coating amount of the coating agent comprising a polyvinyl alcohol copolymer is 3 to 50 parts by weight in terms of the polyvinyl alcohol copolymer relative to 100 parts by weight of the solid formulation containing atorvastatin, a pharmacologically acceptable atorvastatin salt, or a solvate thereof.

5. The atorvastatin-containing coated preparation according to claim 4, wherein the coating amount of the coating agent comprising a polyvinyl alcohol copolymer is 5 to 10 parts by weight in terms of the polyvinyl alcohol copolymer relative to 100 parts by weight of the solid formulation containing atorvastatin, a pharmacologically acceptable atorvastatin salt, or a solvate thereof.

6. The atorvastatin-containing coated preparation according to claim 1, wherein the solid formulation containing atorvastatin, a pharmacologically acceptable atorvastatin salt, or a solvate thereof, is an uncoated tablet or uncoated granule.

7. The atorvastatin-containing coated preparation according to claim 1, wherein the coating agent comprises a polyvinyl alcohol copolymer obtained by copolymerizing a polyvinyl alcohol having an average degree of polymerization of 200 to 1500 and at least one polymerizable vinyl monomer.

8. The atorvastatin-containing coated preparation according to claim 7, wherein the polyvinyl alcohol is a partially saponified polyvinyl alcohol.

9. The atorvastatin-containing coated preparation according to claim 7, wherein the polymerizable vinyl monomer is selected from the group consisting of unsaturated carboxylic acids, unsaturated carboxylic acid esters, unsaturated nitriles, unsaturated amides, aromatic vinyls, aliphatic vinyls, heterocycles having a unsaturated bond, and salts thereof.

10. The atorvastatin-containing coated preparation according to claim 9, wherein the unsaturated carboxylic acids or salts thereof are selected from the group consisting of acrylic acid, methacrylic acid, crotonic acid, fumaric acid, maleic acid, itaconic acid, and salts thereof, and the unsaturated carboxylic acid esters are selected from the group consisting of methyl methacrylate, methyl acrylate, ethyl methacrylate, ethyl acrylate, butyl methacrylate, butyl acrylate, isobutyl methacrylate, isobutyl acrylate, cyclohexyl methacrylate, cyclohexyl acrylate, 2-ethylhexyl methacrylate, 2-ethylhexyl acrylate, hydroxyethyl methacrylate, hydroxyethyl acrylate, polyethylene glycol methacrylate, polyethylene glycol acrylate, and polypropylene glycol acrylate.

11. The atorvastatin-containing coated preparation according to claim 9, wherein the unsaturated carboxylic acids, salts thereof, and unsaturated carboxylic acid esters are compounds represented by general formula (I) or salts thereof:
H₂C=C(R₁)-COOR₂ (I)
wherein R₁ represents a hydrogen atom or a methyl group, and R₂ represents a hydrogen atom or a C₁₋₄ alkyl group.

12. The atorvastatin-containing coated preparation according to claim 10, wherein the unsaturated carboxylic acids or salts thereof are acrylic acid or salts thereof, and the unsaturated carboxylic acid esters are methyl methacrylate.

13. The atorvastatin-containing coated preparation according to claim 7, wherein the polyvinyl alcohol copolymer is obtained by copolymerizing a partially saponified polyvinyl alcohol having an average degree of polymerization of 300 to 500 and a polymerizable vinyl monomer in a weight ratio of 6 : 4 to 9 : 1, the polymerizable vinyl monomer refers to acrylic acid and methyl methacrylate, and the polymerizable vinyl monomer has a weight ratio of about 3 : 7 to about 0.5 : 9.5 when being subjected to copolymerization.

14. A method for inhibiting generation of related substances of atorvastatin, a pharmacologically acceptable atorvastatin salt, or a solvate thereof, the method comprising coating a solid formulation containing atorvastatin, a pharmacologically acceptable atorvastatin salt, or a solvate thereof with a coating agent comprising a polyvinyl alcohol copolymer.

15. A method for stabilizing atorvastatin, a pharmacologically acceptable atorvastatin salt, or a solvate thereof, the method comprising coating a solid formulation containing atorvastatin, a pharmacologically acceptable atorvastatin salt, or a solvate thereof with a coating agent comprising a polyvinyl alcohol copolymer.

## Patentansprüche

1. Ein Atorvastatin enthaltendes beschichtetes Präparat, umfassend eine feste Formulierung, die Atorvastatin, ein pharmakologisch annehmbares Atorvastatinsalz oder ein Solvat davon enthält und die mit einem ein Polyvinylalkoholcopolymer umfassenden Beschichtungsmittel beschichtet ist.

2. Atorvastatin enthaltendes beschichtetes Präparat nach Anspruch 1, wobei eine Atorvastatincalcium oder ein Hydrat davon enthaltende feste Formulierung mit einem Polyvinylalkoholcopolymer umfassenden Beschichtungsmittel beschichtet ist.

3. Atorvastatin enthaltendes beschichtetes Präparat nach Anspruch 1, wobei eine Beschichtungsmenge des Polyvinylalkoholcopolymer umfassenden Beschichtungsmittels 1 bis 100 Gewichtsteile in Form des Polyvinylalkoholcopolymers, bezogen auf 100 Gewichtsteile der Atorvastatin, ein pharmakologisch annehmbares Atorvastatinsalz oder ein Solvat davon enthaltenden festen Formulierung, beträgt.

4. Atorvastatin enthaltendes beschichtetes Präparat nach Anspruch 3, wobei die Beschichtungsmenge des Polyvinylalkoholcopolymer umfassenden Beschichtungsmittels 3 bis 50 Gewichtsteile in Form des Polyvinylalkoholcopolymers, bezogen auf 100 Gewichtsteile der Atorvastatin, ein pharmakologisch annehmbares Atorvastatinsalz oder ein Solvat davon enthaltenden festen Formulierung, beträgt.

5. Atorvastatin enthaltendes beschichtetes Präparat nach Anspruch 4, wobei die Beschichtungsmenge des Polyvinylalkoholcopolymer umfassenden Beschichtungsmittels 5 bis 10 Gewichtsteile in Form des Polyvinylalkoholcopolymers, bezogen auf 100 Gewichtsteile der Atorvastatin, ein pharmakologisch annehmbares Atorvastatinsalz oder ein Solvat davon enthaltenden festen Formulierung, beträgt.

6. Atorvastatin enthaltendes beschichtetes Präparat nach Anspruch 1, wobei die Atorvastatin, ein pharmakologisch annehmbares Atorvastatinsalz oder ein Solvat davon enthaltende feste Formulierung eine unbeschichtete Tablette oder eine unbeschichtete Granalie ist.

7. Atorvastatin enthaltendes beschichtetes Präparat nach Anspruch 1, wobei das Beschichtungsmittel ein Polyvinylalkoholcopolymer umfasst, erhalten durch Copolymerisation eines Polyvinylalkohols mit einem durchschnittlichen Polymerisationsgrad von 200 bis 1500 und mindestens eines polymerisierbaren Vinylmonomers.

8. Atorvastatin enthaltendes beschichtetes Präparat nach Anspruch 7, wobei der Polyvinylalkohol ein partiell verseifter Polyvinylalkohol ist.

9. Atorvastatin enthaltendes beschichtetes Präparat nach Anspruch 7, wobei das polymerisierbare Vinylmonomer aus der aus ungesättigten Carbonsäuren, ungesättigten Carbonsäureestern, ungesättigten Nitrilen, ungesättigten Aminen, aromatischen Vinylverbindungen, aliphatischen Vinylverbindungen, heterocyclischen Verbindungen mit einer ungesättigten Bindung und Salzen davon bestehenden Gruppe ausgewählt ist.

10. Atorvastatin enthaltendes beschichtetes Präparat nach Anspruch 9, wobei die ungesättigten Carbonsäuren oder Salze davon aus der aus Acrylsäure, Methacrylsäure, Crotonsäure, Fumarsäure, Maleinsäure, Itaconsäure und Salzen davon bestehenden Gruppe ausgewählt sind und die ungesättigten Carbonsäureester aus der aus Methylmethacrylat, Methylacrylat, Ethylmethacrylat, Ethylacrylat, Butylmethacrylat, Butylacrylat, Isobutylmethacrylat, Isobutylacrylat, Cyclohexylmethacrylat, Cyclohexylacrylat, 2-Ethylhexylmethacrylat, 2-Ethylhexylacrylat, Hydroxyethylmethacrylat, Hydroxyethylacrylat, Polyethylenglycolmethacrylat, Polyethylenglycolacrylat und Polypropylenglycolacrylat bestehenden Gruppe ausgewählt sind.

11. Atorvastatin enthaltendes beschichtetes Präparat nach Anspruch 9, wobei die ungesättigten Carbonsäuren, Salze davon und die ungesättigten Carbonsäureester Verbindungen sind, die durch die allgemeine Formel (I) dargestellt werden, oder Salze davon:
H₂C=C(R₁)-COOR₂ (I),
worin R₁ ein Wasserstoffatom oder eine Methylgruppe darstellt und R₂ ein Wasserstoffatom oder eine C₁-₄-Alkylgruppe darstellt.

12. Atorvastatin enthaltendes beschichtetes Präparat nach Anspruch 10, wobei die ungesättigten Carbonsäuren oder Salze davon Acrylsäure oder Salze davon und die ungesättigten Carbonsäureester Methylmethacrylat darstellen.

13. Atorvastatin enthaltendes beschichtetes Präparat nach Anspruch 7, wobei das Polyvinylalkoholcopolymer durch Copolymerisieren eines partiell verseiften Polyvinylalkohols mit einem durchschnittlichen Polymerisationsgrad von 300 bis 500 und eines polymerisierbaren Vinylmonomers in einem Gewichtsverhältnis von 6:4 bis 9:1 erhalten wird, sich das polymerisierbare Vinylmonomer auf Acrylsäure und Methylmethacrylat bezieht und das polymerisierbare Vinylmonomer ein Gewichtsverhältnis von etwa 3:7 bis etwa 0,5:9,5 aufweist, wenn sie der Copolymerisation unterworfen werden.

14. Verfahren zur Inhibierung der Bildung von mit Atorvastatin, einem pharmakologisch annehmbaren Atorvastatinsalz oder einem Solvat davon verwandten Substanzen, wobei das Verfahren das Beschichten einer festen Formulierung, die Atorvastatin, ein pharmakologisch annehmbares Atorvastatinsalz oder ein Solvat davon enthält, mit einem Polyvinylalkoholcopolymer umfassenden Beschichtungsmittel umfasst.

15. Verfahren zum Stabilisieren von Atorvastatin, einem pharmakologisch annehmbaren Atorvastatinsalz oder einem Solvat davon, wobei das Verfahren das Beschichten einer festen Formulierung, die Atorvastatin, ein pharmakologisch annehmbares Atorvastatinsalz oder ein Solvat davon enthält, mit einem Polyvinylalkoholcopolymer umfassenden Beschichtungsmittel umfasst.

## Revendications

1. Préparation enrobée contenant de l'atorvastatine comprenant une formulation solide contenant de l'atorvastatine, un sel d'atorvastatine pharmacologiquement acceptable, ou un solvate de celle-ci, enrobée avec un agent d'enrobage comprenant un copolymère d'alcool polyvinylique.

2. Préparation enrobée contenant de l'atorvastatine selon la revendication 1, dans laquelle une formulation solide contenant de l'atorvastatine calcique ou un hydrate de celle-ci est enrobée avec l'agent d'enrobage comprenant un copolymère d'alcool polyvinylique.

3. Préparation enrobée contenant de l'atorvastatine selon la revendication 1, dans laquelle une quantité d'enrobage de l'agent d'enrobage comprenant un copolymère d'alcool polyvinylique est de 1 à 100 parties en poids en termes du copolymère d'alcool polyvinylique par rapport à 100 parties en poids de la formulation solide contenant de l'atorvastatine, un sel d'atorvastatine pharmacologiquement acceptable, ou un solvate de celle-ci.

4. Préparation enrobée contenant de l'atorvastatine selon la revendication 3, dans laquelle la quantité d'enrobage de l'agent d'enrobage comprenant un copolymère d'alcool polyvinylique est de 3 à 50 parties en poids en termes du copolymère d'alcool polyvinylique par rapport à 100 parties en poids de la formulation solide contenant de l'atorvastatine, un sel d'atorvastatine pharmacologiquement acceptable, ou un solvate de celle-ci.

5. Préparation enrobée contenant de l'atorvastatine selon la revendication 4, dans laquelle la quantité d'enrobage de l'agent d'enrobage comprenant un copolymère d'alcool polyvinylique est de 5 à 10 parties en poids en termes du copolymère d'alcool polyvinylique par rapport à 100 parties en poids de la formulation solide contenant de l'atorvastatine, un sel d'atorvastatine pharmacologiquement acceptable, ou un solvate de celle-ci.

6. Préparation enrobée contenant de l'atorvastatine selon la revendication 1, dans laquelle la formulation solide contenant de l'atorvastatine, un sel d'atorvastatine pharmacologiquement acceptable, ou un solvate de celle-ci est un comprimé non enrobé ou un granule non enrobé.

7. Préparation enrobée contenant de l'atorvastatine selon la revendication 1, dans laquelle l'agent d'enrobage comprend un copolymère d'alcool polyvinylique obtenu par copolymérisation d'un alcool polyvinylique ayant un degré moyen de polymérisation de 200 à 1500 et d'au moins un monomère vinylique polymérisable.

8. Préparation enrobée contenant de l'atorvastatine selon la revendication 7, dans laquelle l'alcool polyvinylique est un alcool polyvinylique partiellement saponifié.

9. Préparation enrobée contenant de l'atorvastatine selon la revendication 7, dans laquelle le monomère vinylique polymérisable est choisi dans le groupe constitué par les acides carboxyliques insaturés, les esters d'acide carboxylique insaturés, les nitriles insaturés, les amides insaturés, les vinyles aromatiques, les vinyles aliphatiques, les hétérocycles ayant une liaison insaturée, et les sels de ceux-ci.

10. Préparation enrobée contenant de l'atorvastatine selon la revendication 9, dans laquelle les acides carboxyliques insaturés ou les sels de ceux-ci sont choisis dans le groupe constitué par l'acide acrylique, l'acide méthacrylique, l'acide crotonique, l'acide fumarique, l'acide maléique, l'acide itaconique et les sels de ceux-ci, et les esters d'acide carboxylique insaturés sont choisis dans le groupe constitué par le méthacrylate de méthyle, l'acrylate de méthyle, le méthacrylate d'éthyle, l'acrylate d'éthyle, le méthacrylate de butyle, l'acrylate de butyle, le méthacrylate d'isobutyle, l'acrylate d'isobutyle, le méthacrylate de cyclohexyle, l'acrylate de cyclohexyle, le méthacrylate de 2-éthylhexyle, l'acrylate de 2-éthylhexyle, le méthacrylate d'hydroxyéthyle, l'acrylate d'hydroxyéthyle, le méthacrylate de polyéthylène glycol, l'acrylate de polyéthylène glycol et l'acrylate de polypropylène glycol.

11. Préparation enrobée contenant de l'atorvastatine selon la revendication 9, dans laquelle les acides carboxyliques insaturés, les sels de ceux-ci et les esters d'acide carboxylique insaturés sont des composés représentés par la formule générale (I) ou des sels de ceux-ci :
H₂C=C(R₁)-COOR₂ (I)
dans laquelle R₁ représente un atome d'hydrogène ou un groupe méthyle et R₂ représente un atome d'hydrogène ou un groupe alkyle en C₁ à C₄.

12. Préparation enrobée contenant de l'atorvastatine selon la revendication 10, dans laquelle les acides carboxyliques insaturés ou les sels de ceux-ci sont l'acide acrylique ou des sels de celui-ci, et les esters d'acide carboxylique insaturés sont le méthacrylate de méthyle.

13. Préparation enrobée contenant de l'atorvastatine selon la revendication 7, dans laquelle le copolymère d'alcool polyvinylique est obtenu par copolymérisation d'un alcool polyvinylique partiellement saponifié ayant un degré moyen de polymérisation de 300 à 500 et un monomère vinylique polymérisable selon un rapport pondéral de 6:4 à 9:1, le monomère vinylique polymérisable fait référence à l'acide acrylique et au méthacrylate de méthyle, et le monomère vinylique polymérisable a un rapport pondéral d'environ 3:7 à environ 0,5:9,5 lorsqu'il est soumis à une copolymérisation.

14. Procédé d'inhibition de la production de substances apparentées de l'atorvastatine, d'un sel d'atorvastatine pharmacologiquement acceptable, ou d'un solvate de celle-ci, le procédé consistant à enrober une formation solide contenant de l'atorvastatine, un sel d'atorvastatine pharmacologiquement acceptable, ou un solvate de celle-ci, avec un agent d'enrobage comprenant un copolymère d'alcool polyvinylique.

15. Procédé de stabilisation de l'atorvastatine, d'un sel d'atorvastatine pharmacologiquement acceptable, ou d'un solvate de celle-ci, le procédé consistant à enrober une formulation solide contenant de l'atorvastatine, un sel d'atorvastatine pharmacologiquement acceptable, ou un solvate de celle-ci, avec un agent d'enrobage comprenant un copolymère d'alcool polyvinylique.
